# EUROPÄISCHE PATENTSCHRIFT

(11) **EP 1 135 109 B1**
(45) Veröffentlichungstag und Bekanntmachung des Hinweises auf die Patenterteilung: **02.04.2003**
(21) Anmeldenummer: 99966909.6
(22) Anmeldetag: 30.11.1999
(51) Int. Cl.: A61K 9/16

(54) **VERFAHREN ZUM HERSTELLEN FESTER DARREICHUNGSFORMEN MITTELS SCHMELZEXTRUSION**
METHOD FOR PRODUCING SOLID FORMS OF ADMINISTRATION BY MELT EXTRUSION
PROCEDE DE PRODUCTION DE FORMES DE PRESENTATION SOLIDES PAR EXTRUSION DE MASSE EN FUSION

(30) Priorität: 01.12.1998 DE 19855440
(43) Veröffentlichungstag der Anmeldung: 26.09.2001
(73) Patentinhaber: BASF AKTIENGESELLSCHAFT, 67056 Ludwigshafen (DE)
(72) Erfinder: BERNDL, Gunther, D-67273 Herxheim (DE); KOTHRADE, Stephan, D-67117 Limburgerhof (DE); KESSLER, Thomas, D-67105 Schifferstadt (DE); LANGE, Armin, D-69121 Heidelberg (DE); HOFMANN, Jürgen, D-67069 Ludwigshafen (DE); REINHOLD, Ulrich, D-67346 Speyer (DE)
(74) Vertreter: Kinzebach, Werner, Dr.
(86) Internationale Anmeldenummer: EP9909309
(87) Internationale Veröffentlichungsnummer: WO00032169

(56) Entgegenhaltungen:
- EP-A- 0 729 748
- US-A- 2 802 238
- US-A- 4 176 967
- US-A- 4 889 430
- US-A- 5 350 584

## Beschreibung

Die vorliegende Erfindung betrifft ein Verfahren zum Herstellen fester Darreichungsformen mittels Schmelzextrusion.

Im Gegensatz zu konventionellen Tablettierverfahren, die auf dem Verpressen von Pulvern oder Granulaten beruhen, wird bei der Schmelzextrusion eine wirkstoffhaltige Schmelze eines thermoplastischen, wasserlöslichen oder wasserquellbaren Polymers verarbeitet. Verfahren zum Herstellen von Tabletten und anderen Darreichungsformen mittels Schmelzextrusion sind beispielsweise aus EP-A- 0 240 904, EP-A- 0 240 906, EP-A- 0 337 256, US-A-4 880 585 und EP-A-0 358 105 bekannt.

Man erzeugt dabei ein extrudierbares pharmazeutisches Gemisch durch Mischen und Aufschmelzen eines polymeren Bindemittels, wenigstens eines pharmazeutischen Wirkstoffs und gegebenenfalls weiterer Additive. Zum Mischen und Aufschmelzen wird üblicherweise ein Extruder verwendet. Die einzelnen Komponenten können jedoch auch vor dem Einfüllen in den Extruder vermischt werden. Die wirkstoffhaltige Schmelze wird über eine oder mehrere Düsen, beispielsweise Schlitzdüsen im Extruderkopf in Form von Produktsträngen oder -bändern ausgepreßt. Die noch plastischen Produktstränge oder -bänder werden dann mit Hilfe geeigneter Werkzeuge zu Tabletten oder anderen Darreichungsformen, wie Zäpfchen oder Granulaten, geformt. Beispielsweise kann die extrudierte Schmelze durch ein Kalandrierverfahren mittels gegenläufig rotierender Formwalzen zu der gewünschten Darreichungsform verpreßt werden. Dabei sind in einer oder in beiden Formwalzen Vertiefungen mit zu der gewünschten Tablette oder dem Zäpfchen komplementärer Form vorgesehen. Gemäß einer anderen bekannten Variante läßt man zwischen glatten Kalanderwalzen ein Band hindurchlaufen, das Vertiefungen oder Öffnungen in der gewünschten Tabletten- oder Zäpfchenform aufweist.

Als Extruder werden üblicherweise Einschnecken- oder Doppelschneckenextruder verwendet. In dem europäischen Patent EP-B- 0 580 860 wird ein Verfahren zur Herstellung von festen pharmazeutischen Dispersionen beschrieben, bei dem ein Doppelschneckenextruder verwendet wird, der Knetscheiben aufweist. In der europäischen Patentanmeldung EP-A- 0 729 748 wird der Einsatz von Mehrwellenextrudern bei der Herstellung von pharmazeutischen Zusammensetzungen erwähnt. Tatsächlich behandelt dieses Dokument jedoch nur Doppelschneckenextruder mit Knetscheiben. Extruder mit mehr als zwei Schnecken werden weder in EP-B- 0 580 860 noch in EP-A- 0 729 748 beschrieben.

Derartige Doppelschneckenextruder weisen jedoch den Nachteil auf, daß im Bereich der Knetscheiben punktuell auftretende Temperaturspitzen und hohe Scherbelastungen auf die zu plastifizierende Masse einwirken. Dies stellt vor allem für die Extrusion von wirkstoffhaltigen Schmelzen ein Problem dar, da zahlreiche Wirkstoffe äußerst wärmeempfindlich sind. Außerdem können bei herkömmlichen Doppelschneckenextrudern mit Knetscheiben nur solche polymere Bindemittel und Additive verwendet werden, die unempfindlich gegenüber erhöhten Temperaturen und hoher Scherbelastung sind. Diese Nachteile schränken die Bandbreite der herkömmlicherweise bei der Tablettenherstellung mittels Schmelzextrusion verwendbaren Substanzen stark ein.

Aufgabe der vorliegenden Erfindung ist es daher ein Verfahren zum Herstellen von festen Darreichungsformen mittels Schmelzextrusion bereitzustellen, das es ermöglicht, die Ausgangssubstanzen des extrudierbaren Gemisches, also insbesondere das polymere Bindemittel und die pharmazeutischen Wirkstoffe in schonender Weise, insbesondere ohne Auftreten hoher Scher- und Temperaturbelastungen zu plastifizieren und zu vermischen.

Gelöst wird diese Aufgabe durch das Verfahren gemäß beigefügtem Hauptanspruch. Erfindungsgemäß wird zum Herstellen von festen Darreichungsformen mittels Schmelzextrusion vorgeschlagen, ein polymeres Bindemittel, wenigstens einen pharmazeutischen Wirkstoff und gegebenenfalls weitere Additive in einem Planetwalzenextruder zu mischen, aufzuschmelzen und anschließend in Form eines kontinuierlichen, plastischen Produktstranges zu extrudieren.

Überraschend wurde gefunden, daß es bei Verwendung eines Planetwalzenextruders möglich ist, auch empfindliche Polymere, Werkstoffe und Additive in feste Darreichungsformen einzuarbeiten.

Planetwalzenextruder sind an sich bekannt und werden beispielsweise in Deutschland von der Firma Entex Rust & Mitschke GmbH, Bochum, hergestellt.

Planetwalzenextruder sind kontinuierliche Schneckenkneter, deren Knetteil nach Art eines Planetenwalzwerkes ausgebildet ist. Wie herkömmliche Ein- oder Doppelschneckenextruder besitzen auch Planetwalzenextruder einen Materialeinzug, an den sich eine Plastifizierungs- und Homogenisierungszone anschließt. Üblicherweise ist vor der Austrittsdüse eine Kühlzone angeordnet, um das erhitzte Materialgemisch auf Extrusionstemperatur abzukühlen. Die Plastifizierungs- und Homogenisierungszone weist eine zentrale Spindel auf, die typischerweise unter 45o verzahnt ist. Mit dieser Zentralspindel kämmen mehrere Planetspindeln, die ihrerseits wieder in eine innen verzahnte, zylindrische Buchse eingreifen. Wird die Zentralspindel angetrieben, so laufen die Planetenspindeln in einem Abwälzvorgang zwischen Buchse und Zentralspindel frei um. Sie sind nicht gelagert und schwimmen während des Betriebs in der zu extrudierenden Masse. Jede Planetspindel stellt, soweit sich die Zähne mit der Zentralspindel bzw. der Innenverzahnung der Buchse in Eingriff befinden, eine Art Schraubenpumpe dar. Die zu plastifizierende Masse wird typischerweise axial in die Plastifizierungs- und Homogenisierungszone des Planetwalzenextruders eingeschoben und zwischen den umlaufenden Planetspindeln und der Zentralspindel einerseits bzw. der innenverzahnten Buchse andererseits sehr stark ausgewalzt.

Die zu plastifizierende Masse, die in das Spaltspiel der Verzahnung gelangt, wird dabei immer wieder einer kurzzeitigen punktförmigen Walzbeanspruchung unterworfen, aber aufgrund der abrollenden Bewegung der Planetspindeln auf der Zentralspindel sofort wieder entspannt und freigelegt. Aufgrund dieser Dünnschichtverwalzung nimmt die Masse in sehr kurzer Zeit die erforderliche Plastifizierwärme auf und wird intensiv gemischt und geknetet und dabei homogenisiert.

Aufgrund ihres gegenüber Ein- und Doppelschneckenextrudern wesentlich höheren Wirkungsgrades bauen Planetwalzenextruder üblicherweise sehr kurz, so daß auch die Verweilzeiten der zu plastifizierenden Masse in der Plastifizierungs- und Homogenisierungszone sehr kurz sind.

Das plastifizierte und homogenisierte Material wird ggf. von einer nachgeschalteten kurzen Austragsschnecke erfaßt und kann durch Lochplatten oder sonstige Düsen extrudiert werden. Der Extruder kann jedoch auch ohne Düsenplatte ohne Druckaufbau betrieben werden.

Üblicherweise ist die Plastifizierungs- und Homogenisierungszone des Extruders temperierbar. Dazu können beispielsweise Heiz- oder Kühlmittel durch den die Buchse umgebenden Gehäusemantel des Extruders geführt werden.

Besonders vorteilhaft ist der Planetwalzenextruder modulartig aus einzelnen Abschnitten aufgebaut, wobei die Auslegung der Walzen und das Temperaturprofil für jeden Abschnitt separat optimiert werden kann.

Der Planetwalzenextruder zeichnet sich auch durch eine gute Selbstreinigung aus, was insbesondere bei der Herstellung von pharmazeutischen Erzeugnissen vorteilhaft ist.

Die zwischen den Walzwerken vorhandenen drucklosen Kammern gewährleisten eine gute Entgasung der zu plastifizierenden Masse.

Mit dem erfindungsgemäßen Verfahren ist es trotz der nur kurzzeitigen Temperatur- und Scherbeanspruchung möglich, eine optimale Homogenisierung der Masse in kürzester Zeit zu erreichen. Bei der Herstellung von festen Lösungen erweist sich dies als vorteilhaft, da es gelingt, ohne Verwendung von Lösungsmitteln und hohen Temperaturen eine molekulardisperse Verteilung des Wirkstoffes in der Matrix zu erreichen.

Durchschnittlich liegen die zur Plastifizierung und Homogenisierung des pharmazeutisches Gemisches notwendigen Temperaturen mit dem erfindungsgemäßen Verfahren, d.h. bei Verwendung eines Planetwalzenextruders, um ca. 20 °C niedriger als die mit herkömmlichen Extrusionsverfahren, d.h. bei Verwendung eines Zweischneckenextruders mit Knetscheiben, erforderlichen Temperaturen.

Vorteilhaft verwendet man einen Planetwalzenextruder der eine Zentralspindel und drei bis acht Planetspindeln aufweist.

Besonders bevorzugt verwendet man einen Planetwalzenextruder mit sechs Planetspindeln. Mit dieser Anordnung kann man das zu extrudierende Gemisch besonders effektiv vermischen und plastifizieren, ohne daß eine übermäßige Temperatur- und Scherbelastung des Materials auftritt.

Aufgrund der guten Durchmischung und Plastifizierung des pharmazeutischen Gemisches benötigt der Planetwalzenextruder keine Knetscheiben. Die oben beschriebenen Nachteile, die etwa beim Einsatz von Doppelschneckenextrudern mit Knetscheiben auftreten, werden mit dem erfindungsgemäßen Verfahren vermieden.

Die Verweilzeit des pharmazeutischen Gemisches in dem Planetwalzenextruder ist kurz und beträgt bevorzugt, abhängig von der Drehzahl der Zentralspindel und der Länge des Walzenteils, etwa 0,5 bis 2 Minuten.

Da mit dem erfindungsgemäßen Verfahren die zu plastifizierende Masse keinen länger anhaltenden Temperatur- und Scherbeanspruchungen ausgesetzt ist, eignet sich ein Planetwalzenextruder insbesondere zum Extrudieren von Massen, die wärme- oder scherempfindliche Substanzen enthalten, wobei es sich dabei um Wirk-, Hilfs- oder Zusatzstoffe handeln kann.

Gegenstand der vorliegenden Erfindung ist daher auch die Verwendung eines Planetwalzenextruders zum Extrudieren eines wärmeempfindlichen pharmazeutischen Gemisches.

Der Begriff "Darreichungsform" im Sinne der vorliegenden Erfindung ist breitest möglich zu verstehen. Er ist weder an eine bestimmte Form, noch eine bestimmte Anwendung gebunden. Er umfaßt daher beispielsweise Tabletten zur peroralen Anwendung, Zäpfchen zur rektalen Anwendung, Pellets, Granulate oder auch größere Formen, wie Würfel, Blöcke (Quader) oder zylindrische Formen. Das erfindungsgemäße Verfahren ist zur Herstellung von beliebigen Darreichungsformen geeignet, die etwa als Arzneimittel, Pflanzenbehandlungsmittel, Futtermittel und Nahrungsmittel, sowie zur Abgabe von Riechstoffen und Parfümölen Verwendung finden.

Unter pharmazeutischen Wirkstoffen im Sinne der Erfindung sind alle Stoffe mit einer pharmazeutischen Wirkung und möglichst geringen Nebenwirkungen zu verstehen, so fern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Einzige Bedingung ist dabei, daß sie zur Erzielung der gewünschten Wirkung ausreichen. So kann die Wirkstoffkonzentration, bezogen auf das Gesamtgewicht der Darreichungsform, im Bereich von 0 bis 90, vorzugsweise von 0,1 bis 60 liegen. Der Begriff Wirkstoff umfaßt im vorliegenden Zusammenhang auch beliebige Wirkstoffkombinationen. Wirkstoffe im Sinne der Erfindung sind beispielsweise auch Vitamine. Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin und Captopril.

Das erfindungsgemäße Verfahren eignet sich aber insbesondere für wärmeempfindliche Substanzen, wie beispielsweise Enzyme, Peptide, Vitamine, Hormone, Insulin, Pflanzenextrakte, Dihydropyridinderivate, Antibiotika, wie beispielsweise Makrolyte oder Zytostatika. Besonders geeignet ist das erfindungsgemäße Verfahren auch zur Extrusion von Pflanzenextrakten und anderen natürlichen Wirkstoffen.

Das erfindungsgemäße Verfahren eignet sich insbesondere zur Herstellung von festen Darreichungsformen, die solche Polymere enthalten, die aufgrund ihres hohen Molekulargewichts oder ihrer Thermolabilität bei der Extrusion in Zweischneckenextrudern Abbauerscheinungen unterliegen, beispielsweise einem oxidativen Abbau, Depolymerisation, Molekulargewichtsabbau, Eliminierung von Seitengruppen, oder die chemische Reaktionen mit anderen Komponenten der Formulierung eingehen.

Das polymere Bindemittel muß in der Gesamtmischung aller Komponenten im Bereich von 50 bis 250 °C, vorzugsweise 60 bis 180 °C und besonders bevorzugt im Bereich von 80 bis 150 °C erweichen oder schmelzen. Die Glasübergangstemperatur des Gemisches muß daher unter 200 °C, vorzugsweise unter 150 °C und besonders bevorzugt unter 130 °C liegen. Erforderlichenfalls wird sie durch übliche, pharmakologisch akzeptable weichmachende Hilfsstoffe herabgesetzt. Geeignete polymere Bindemittel sind beispielsweise beschrieben in WO 97/15291.

Als polymere Bindemittel werden für die Schmelzextrusion pharmazeutischer Wirkstoffe bevorzugt eingesetzt: Polymere oder Copolymerisate von N-Vinylpyrrolidon, Eudragittypen (Acrylharze) oder Cellulosen. Dabei sind besonders bevorzugt: Polyvinylpyrrolidon (PVP), Copolymerisate von N-Vinylpyrrolidon und Vinylestern, wie Vinylacetat, Poly(hydroxyalkylacrylate), Poly(hydroxyalkylmethacrylate), Polyacrylate, Polymethacrylate, Alkylcellulosen oder Hydroxyalkylcellulosen.

Das extrudierbare Gemisch kann neben dem polymeren Bindemittel und dem (oder den) Wirkstoff(en) auch übliche Zusätze enthalten, beispielsweise Weichmacher, Schmiermittel, Fließmittel, Farbstoffe, Stabilisatoren oder Netz-, Konservierungs-, Spreng- Adsorptions-. Formentrenn- und Treibmittel. Ebenso können übliche galenische Hilfsmittel, z.B. Streckmittel bzw. Füllstoffe enthalten sein. Geeignete Zusätze und galenische Hilfsmittel sind beispielsweise beschrieben in WO 97/15291.

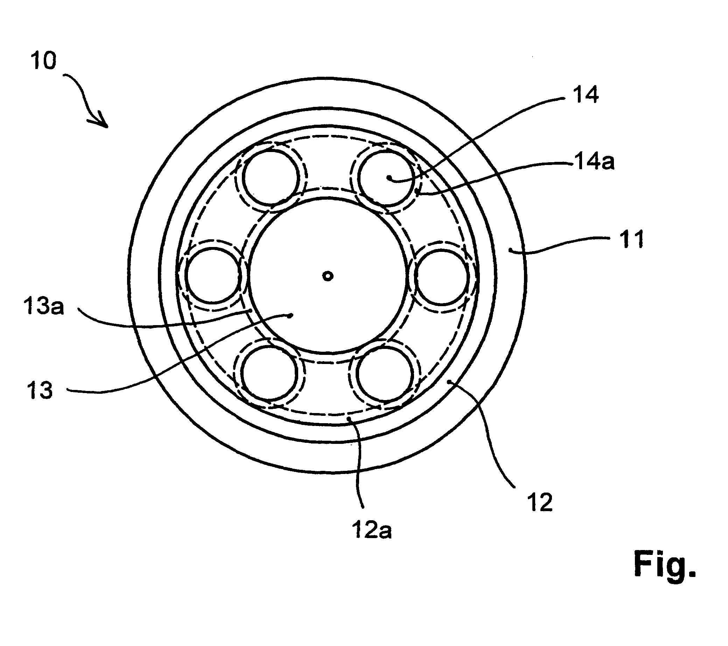

Der typische Aufbau eines Planetwalzenextruders ist in der Schnittansicht der beigefügten Figur dargestellt.

Der Planetwalzenextruder 10 weist in seinem Plastifizierungs- und Homogenisierungsbereich einen temperierbaren, im wesentlichen zylindrischen Gehäusemantel 11 auf, an dessen Innenwand eine Buchse 12 angeordnet ist, auf deren Innenfläche eine spiralförmige Nut 12a ausgespart ist. Zentral im Inneren der Buchse 12 ist eine antreibbare Zentralspindel 13 drehbar gelagert, die von mehreren frei umlaufenden Planetenspindeln 14 umgeben ist. Jede Planetenspindel 14 kämmt mit ihrer spiralförmigen Mantelfläche 14a sowohl mit der spiralförmigen Mantelfläche 13a der Zentralspindel 13 als auch mit der Innenspirale 12a der Buchse 12. Die Buchse 12 ist drehfest im Gehäuse des Planetwalzenextruders 10 angeordnet. Für die umlaufenden Planetenspindeln 14 ist am Extruderende ein in der Schnittdarstellung der beigefügten Figur nicht erkennbarer Anlaufring angeordnet.

### Beispiel 1:

Herstellung einer festen Lösung von Ibuprofen in einer Matrix aus Kollidon® 90 F unter Verwendung eines Planetwalzenextruders
30 Gew.% Ibuprofen wurden mit 69,5 Gew.% Kollidon® 90 F mit einem k Wert von 90 und 0,5 Gew. % Aerosil 200 in einem Planetwalzenextruder extrudiert.
Der Planetwalzenextruder hatte eine Zentralspindel mit einem Durchmesser von 43 mm, die von sechs Planetspindeln mit Durchmessern von jeweils 20 mm und einer Länge von jeweils 398 mm umgeben war.Bei einer Drehzahl von 40 U/min wurden mit einem Durchsatz von 5 kg/h extrudiert.
Die Plastifizierung und Homogenisierung erfolgte bei einer maximalen Temperatur im Extruder von 150 °C.
Der k Wert von Kollidon betrug nach der Extrusion 85.

### Vergleichsbeispiel 1:

Herstellung einer festen Lösung von Ibuprofen in einer Matrix aus Kollidon® 90 F unter Verwendung eines Doppelschneckenextruders
30 Gew.% Ibuprofen wurden mit 69,5 Gew.% Kollidon® 90 F mit einem k Wert von 90 und 0,5 Gew. % Aerosil 200 in einem ZSK-Zweiwellenextruder der Fa. Werner & Pfleiderer bei einer Drehzahl von 100 U/min und einem Durchsatz von 2 kg/h extrudiert.
Für eine zufriedenstellende Plastifizierung und Homogenisierung war eine maximale Temperatur im Extruder von 190 °C erforderlich.
Der k Wert von Kollidon betrug nach der Extrusion nur noch etwa 70.

## Patentansprüche

1. Verfahren zum Herstellen von festen Darreichungsformen mittels Schmelzextrusion, wobei man ein polymeres Bindemittel, wenigstens einen pharmazeutischen Wirkstoff und gegebenenfalls weitere Additive in einem Extruder mischt und aufschmelzt und anschließend einen kontinuierlichen, plastischen Produktstrang extrudiert, **dadurch gekennzeichnet, daß** man als Extruder einen Planetwalzenextruder (10) verwendet.

2. Verfahren gemäß Anspruch 1, **dadurch gekennzeichnet, daß** man einen Planetwalzenextruder (10) mit einer Zentralspindel (13) und drei bis acht Planetspindeln (14) verwendet.

3. Verfahren gemäß Anspruch 2, **dadurch gekennzeichnet, daß** man einen Planetwalzenextruder (10) mit sechs Planetspindeln (14) verwendet.

4. Verfahren gemäß einem der Ansprüche 1 bis 3, **dadurch gekennzeichnet, daß** man einen Planetwalzenextruder (10) ohne Knetscheiben verwendet.

5. Verfahren gemäß einem der Ansprüche 2 bis 4, **dadurch gekennzeichnet, daß** man die Drehzahl der Zentralspindel (14) des Planetwalzenextruders (10) so einstellt, daß die Verweilzeit eines zu extrudierenden pharmazeutischen Gemisches in dem Extruder (10) etwa 0,5 bis 2 Minuten beträgt.

6. Verwendung eines Planetwalzenextruders zum Extrudieren eines wärme- und/oder scherempfindlichen pharmazeutischen Gemisches.

## Revendications

1. Procédé pour la préparation de formes de présentation solides par extrusion de masse en fusion, dans lequel on mélange et on fait fondre dans une extrudeuse un liant polymère, au moins une substance à activité pharmaceutique et éventuellement d'autres additifs, et on extrude ensuite un boudin de produit plastique en continu, **caractérisé par le fait qu'**on utilise comme extrudeuse une extrudeuse à cylindres planétaires (10).

2. Procédé selon la revendication 1, **caractérisé par le fait qu'**on utilise une extrudeuse à cylindres planétaires (10) ayant une broche centrale (13) et trois à huit broches planétaires (14).

3. Procédé selon la revendication 2, **caractérisé par le fait qu'**on utilise une extrudeuse à cylindres planétaires (10) ayant six broches planétaires (14).

4. Procédé selon l'une des revendications 1 à 3, **caractérisé par le fait qu'**on utilise une extrudeuse à cylindres planétaires (10) sans disque de malaxage.

5. Procédé selon l'une des revendications 2 à 4, **caractérisé par le fait qu'**on ajuste la vitesse de rotation de la broche centrale (14) de l'extrudeuse à cylindres planétaires (10) de telle manière que la durée de séjour d'un mélange pharmaceutique à extruder dans l'extrudeuse (10) soit d'environ 0,5 à 2 minutes.

6. Utilisation d'une extrudeuse à cylindres planétaires pour l'extrusion d'un mélange pharmaceutique sensible à la chaleur et/ou au cisaillement.

## Claims

1. A method for producing solid forms of administration by melt extrusion, wherein a polymeric binder, at least one active pharmaceutical agent and, if required, further additives are mixed in an extruder and melted, and are subsequently extruded in a continuous ductile production string, **characterized in that** a planetary roller extruder (10) is used as the extruder.

2. The method in accordance with claim 1, **characterized in that** a planetary roller extruder (10) with a central spindle (13) and three to eight planetary spindles (14) is used.

3. The method in accordance with claim 2, **characterized in that** a planetary roller extruder (10) with six planetary spindles (14) is used.

4. The method in accordance with one of claims 1 to 3, **characterized in that** a planetary roller extruder (10) without kneading disks is used.

5. The method in accordance with one of claims 2 to 4, **characterized in that** the number of revolutions of the central spindle (14) of the planetary roller extruder (10) is set in such a way that the dwell time in the extruder (10) of a pharmaceutical mixture to be extruded is approximately 0.5 to 2 minutes.

6. Use of a planetary roller extruder for extruding a heat- and/or shear-sensitive pharmaceutical mixture.
